# EUROPEAN PATENT SPECIFICATION

(11) **EP 3 653 231 B1**
(45) Date of publication and mention of the grant of the patent: **14.06.2023**
(21) Application number: 19207791.5
(22) Date of filing: 07.11.2019
(51) Int. Cl.: A61L 9/12, A61L 9/14, G06K 19/00, H04B 5/00

(54) **AROMA CARTRIDGE AND AROMA DISPLAY**
AROMAPATRONE UND AROMAANZEIGE
CARTOUCHE D'ARÔME ET AFFICHAGE D'ARÔME

(30) Priority: 12.11.2018 JP 2018212000
(43) Date of publication of application: 20.05.2020
(73) Proprietor: Aromajoin Corporation, Kyoto-city, Kyoto 612-8374 (JP)
(72) Inventor: NGUYEN, Quang Van, Kyoto 6128374 (JP); KIM, Dong Wook, Kyoto 6128374 (JP)
(74) Representative: Treeby, Philip David William

(56) References cited:
- WO-A1-2016/098114
- WO-A1-2017/062485
- JP-A- 2014 230 632
- US-A1- 2014 001 286
- US-A1- 2018 036 448

## Description

### FIELD OF THE INVENTION

The present invention relates to an aroma display and, more specifically to an improvement of an aroma display that is capable of emission of various scents by loading a plurality of aroma cartridges. BACKGROUND OF THE INVENTION

Human communication is done in various modes based on human senses. Most frequently used are visual and auditory communications. By contrast, olfaction or sense of smell, on which we rely considerably in our lives, is hardly used for communication. However, if the sense of smell can be utilized in addition to visual and auditory senses, communication would be more effective and various people would be able to share deeper experiences.

Focusing on this point, recently, devices have been used which are used with audio-visual reproducing devices such as television receivers (hereinafter referred to as TVs), personal computers (hereinafter referred to as PCs) and game machines for generating scents appropriate for the time and place. In the present Specification, such a device that generates scent appropriate for scenes will be referred to as an aroma display.

Emission of a certain scent supposed to be fit for a certain scene would not be very effective if there is any lingering scent produced immediately before. Therefore, an aroma display is desirable that can diffuse a desired scent in a certain limited scope at one time point and can blow out that scent and switch to another scent at another time point.

Without the capability to freely switch from one scent to another, an aroma display cannot make full use of scents. For this purpose, one possible approach is to prepare a plurality of cartridges (referred to as aroma cartridges) each containing a pre-selected scent emission source (referred to as a scent source), to load them to an aroma display and to cause the desired cartridge to emit the scent.

A scent channel for emitting the scent from the scent source in the cartridge is formed in the cartridge, and a mechanism for feeding air into the cartridge is provided to the aroma display, so that the scent is emitted through the channel to the outside. Aside from the mechanism for emitting scent from each cartridge, an air emitting mechanism for emitting air free of any scent component, having an air emission opening near the scent passage, is provided in the aroma display. For switching scents after one scent is emitted, air is emitted from the air emitting mechanism to blow out the scented air therearound and then another scent is emitted. Thus, scents can be switched at any timing, avoiding improper blending of scents.

Such a technique is described in JP2014-092673A. The technique disclosed in this reference is as described above. It is noted, however, that an opening is formed in the housing of an aroma display for emitting the scent to the outside, and the scent channels from respective aroma cartridges and air emission openings are all connected to this opening. Each aroma cartridge is detachably loaded to the housing of the aroma display. An opening for introducing air into the aroma cartridge is formed at a prescribed portion of each aroma cartridge. At that portion of the aroma display which corresponds to this opening when an aroma cartridge is loaded on the aroma display, a wind source containing a diaphragm with a piezoelectric element attached is mounted corresponding to each aroma cartridge. By applying AC voltage to the piezoelectric element, the diaphragm vibrates and introduces air into the aroma cartridge through a nozzle provided on the wind source and through the opening of the aroma cartridge. From the aroma cartridge that received the introduced air, scented air corresponding to the scent source therein is emitted to the outside through the scent channel.

JP 2014 230632 A discloses an olfactory display and fragrance source cartridge used therefor including a housing and presents a fragrance within a range bounded in terms of time and space. In the housing, a plurality of fragrance chambers partitioned with a diaphragm are formed, and an air inlet and a fragrance outlet are formed in each fragrance chamber. Each fragrance outlet is communicated with an emitting port via a fragrance channel. Additionally, in each fragrance chamber, a solid-like fragrance source is accommodated and an airflow source to send air into the fragrance chamber from an air inlet is installed. Furthermore, in the housing, an auxiliary airflow source to accelerate a fragrance sent from the fragrance chamber by the airflow source in a fragrance emission direction is installed apart from the airflow source.

US 2014/001286 discloses a fragrance dispensing device includes a housing, a fragrance assembly, a cover panel, a fan, and a drive assembly. The housing includes a first portion and a second portion, which moves relative to the first portion. The housing includes an intake opening and an exhaust opening. The fragrance assembly rotates within the housing's interior cavity and defines a plurality of receptacles for receiving fragrance inserts. The cover panel substantially seals one or more receptacles and defines an opening. The fan is coupled to the housing's second portion. The drive assembly rotates the fragrance assembly such that at least a portion of a user-selected fragrance insert is aligned with the cover panel's opening. The fan moves air from the housing's intake opening, across the cover panel's opening, and out the housing's exhaust opening, thereby releasing a fragrance into an environment. The drive assembly may include a shaft with helical threads.

### SUMMARY OF THE INVENTION

### Problems to be Solved by the Invention

The aroma display having the above-described structure is capable of freely switching scents by selecting the wind source or sources to be operated. Further, by operating the air emitting mechanism at that time, it is possible to prevent undesired blending of scents. The wind source to be operated and its timing may be controlled by sending an external command to the aroma display. Therefore, the aroma display can achieve magnificent effects of emitting desired scents appropriately by causing desired wind sources at desired timings of movies or animation films.

Here, the information as to which wind source is to be operated to emit which scent is indispensable to enable emission control for emitting desired scents. For this purpose, according to JP2014-092673A, identification numbers are allocated to the aroma cartridge loading sections of the aroma display. An aroma display having a capacity of six cartridges as shown as an example in JP2014-092673A has numbers 1 to 6 allocated to the respective cartridge loading sections. Information indicating what type of scent comes from which cartridge loaded to the section of which number is recorded in a controller (PC or the like) that controls the aroma display. To identify various different scents, each aroma cartridge has an ID code indicating its scent. Every time an aroma cartridge is loaded to the aroma display, the user inputs the number of the loading section and the ID code of the aroma cartridge loaded to the loading section associated with each other.

This user must repeat this process every time he/she changes the aroma cartridges. This is a troublesome task prone to input error. Erroneous input leads to emission of a scent not intended, making the valuable communication tool useless or spoiling the communication. This problem becomes more serious as the number of cartridges loadable to the aroma display increases and the number of scents of the aroma cartridges increases.

Therefore, it is preferable to provide an aroma cartridge and an aroma display enabling reliable control of aroma cartridges and reducing the possibility of erroneous emissions of the scent not intended.

### Means to Solve the Problems

According to a first aspect, the present invention provides an aroma cartridge, including a columnar housing having first and second surfaces each substantially having a shape of a triangle parallel to and congruent with each other and a side surface connecting the first and second surfaces; wherein said housing has a hollow portion to store a scent source, a scent passage opening at said first surface for emitting scent from the scent source in the hollow portion to the outside, connecting said hollow portion to the outside, and an opening for introducing air from outside to said hollow portion; each of said first and second surfaces has a corresponding first vertex; said scent passage is opened at a portion near said first vertex of said first surface; said aroma cartridge further comprising a near field communication tag provided on said second surface or said side surface of said housing for transmitting identification data for identifying type of scent source contained in the aroma cartridge to a near field communication chip by near field communication; wherein said near field communication tag is arranged on the surface of said housing, at a position closer to said second surface than said first surface and closer to an opposite side of said first vertex of said second surface than said first vertex of said second surface, said opening is formed on said side surface, and said near field communication tag is arranged at a position closer to the center of said opposite side than said first vertex on said second surface of said housing.

Preferably, the triangle is an isosceles triangle and the first vertex is the vertex formed by two sides of equal length of the isosceles triangle.

More preferably, the triangle is an equilateral triangle.

More preferably, the near field communication tag is attached to the housing by an adhesive sticker.

According to a second aspect, the present invention provides an aroma display having a cartridge loading section for containing a plurality of aroma cartridges, for emitting scent from each of said plurality of aroma cartridges; wherein each of said plurality of aroma cartridges includes a columnar housing having first and second surfaces each substantially having a shape of a triangle parallel to and congruent with each other and a side surface connecting the first and second surfaces; said housing has a hollow portion to store a scent source, a scent passage opening at said first surface for emitting scent from the scent source in the hollow portion to the outside, connecting said hollow portion to the outside, and an opening for introducing air from outside to said hollow portion; each of said first and second surfaces has a corresponding first vertex; said scent passage is opened at a portion near said first vertex of said first surface; said aroma cartridge further includes a near field communication tag provided on said second surface or said side surface of said housing for transmitting identification data for identifying type of scent source contained in the aroma cartridge to a near field communication chip by near field communication; said near field communication tag is arranged on the surface of said housing, at a position closer to said second surface than said first surface and closer to an opposite side of said first vertex of said second surface than said first vertex of said second surface; wherein said opening is formed on said side surface, and said near field communication tag is arranged at a position closer to the center of said opposite side than said first vertex on said second surface of said housing; said cartridge loading section of said aroma display has an inner shape rotationally symmetric about a prescribed center, and receives each said aroma cartridge such that said first vertex is positioned adjacent to said center and such that said second surfaces of said plurality of aroma cartridges are aligned on one plane; said aroma display further comprising a chip stage mounting a plurality of near field communication chips arranged at positions enabling communication with said near field communication tag of any of said plurality of aroma cartridges, respectively corresponding to said plurality of aroma cartridges loaded in said cartridge loading section, wherein each of said plurality of near field communication chips is arranged at a position closer to the center of the opposite side of said first vertex than said first vertex of said second surface of one of said plurality of aroma cartridges.

Preferably, in the aroma display, each of the near field communication chips receives, when the aroma cartridge is loaded at a portion close to the near field communication chip, data identifying the scent source contained in the aroma cartridge by near field communication with the near field communication tag of the aroma cartridge, and outputs the data identifying the scent source and data identifying the near field communication tag. The aroma display further includes a control circuit receiving the data identifying the scent source from each of the near field communication chips, and based on the data, driving that aroma cartridge which has a desired scent source to emit the scent.

More preferably, the plurality of near field communication chips are arranged on a circle centered at the prescribed center.

More preferably, the plurality of near field communication chips are arranged on the circle at an equal distance from each other.

Preferably, the circle is on the side opposite to the first surface with respect to the plane on which the second surfaces are aligned.

According to a third aspect, the present invention provides an aroma display system one or a plurality of aroma cartridges and an aroma display emitting scent from the plurality of aroma cartridges; wherein each of said plurality of aroma cartridges includes a columnar housing having first and second surfaces each substantially having a shape of a triangle parallel to and congruent with each other and a side surface connecting the first and second surfaces; said housing has a hollow portion to store a scent source, a scent passage opening at said first surface for emitting scent from the scent source in the hollow portion to the outside, connecting said hollow portion to the outside, and an opening for introducing air from outside to said hollow portion; each of said first and second surfaces has a corresponding first vertex; said scent passage is opened at a portion near said first vertex of said first surface; said aroma cartridge further includes a near field communication tag provided on said second surface or said side surface of said housing for transmitting identification data for identifying type of scent source contained in the aroma cartridge to a near field communication chip by near field communication; said near field communication tag is arranged on the surface of said housing, at a position closer to said second surface than said first surface and closer to an opposite side of said first vertex of said second surface than said first vertex of said second surface; wherein said opening is formed on said side surface, and said near field communication tag is arranged at a position closer to the center of said opposite side than said first vertex on said second surface of said housing; said aroma display has a cartridge loading section for containing a plurality of aroma cartridges; said cartridge loading section has an inner shape rotationally symmetric about a prescribed center, and receives each said aroma cartridge such that said first vertex is positioned adjacent to said center and such that said second surfaces of said plurality of aroma cartridges are aligned on one plane; said aroma display further includes a chip stage mounting a plurality of near field communication chips arranged at positions enabling communication with said near field communication tag of any of said plurality of aroma cartridges, respectively corresponding to said plurality of aroma cartridges loaded in said cartridge loading section, and wherein each of said plurality of near field communication chips is arranged at a position closer to the center of the opposite side of said first vertex than said first vertex of said second surface of one of said plurality of aroma cartridges.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 shows an appearance of the aroma display in use, in accordance with an embodiment of the present invention.
Fig. 2 is a perspective view from obliquely above of the aroma display shown in Fig. 1.
Fig. 3 is a perspective view from obliquely below of the aroma display shown in Fig. 1.
Fig. 4 is a cross-section of a plane 4-4 of the aroma display shown in Fig. 1.
Fig. 5 is an exploded perspective view from obliquely above of the aroma display shown in Fig. 2.
Fig. 6 is an exploded perspective view from obliquely below of the aroma display shown in Fig. 3.
Fig. 7 is an enlarged perspective view of a portion indicated by reference number 7 in Fig. 5.
Fig. 8 is a plan view of an NFC chip stage.
Fig. 9 is a perspective view from obliquely above of the aroma cartridge.
Fig. 10 is a perspective view from obliquely above of the aroma cartridge turned upside-down.
Fig. 11 is a cross-section of the aroma cartridge shown in Figs. 2 and 4 taken along 11-11.
Fig. 12 is an enlarged view of the portion indicated by reference number 12 in Fig. 11.
Fig. 13 is an enlarged cross-section of a micro-blower.
Fig. 14 is a cross-section showing routes of air flow in the aroma display.

### DESCRIPTION OF THE PREFERRED EMBODIMENTS

In the following description and in the drawings, the same components are denoted by the same reference characters. Therefore, detailed description thereof will not be repeated. It is naturally understood that not every components of the embodiments described below is indispensable for the present invention. Some of the components may be omitted or replaced by other forms.

### [First Embodiment]

### <Structure>

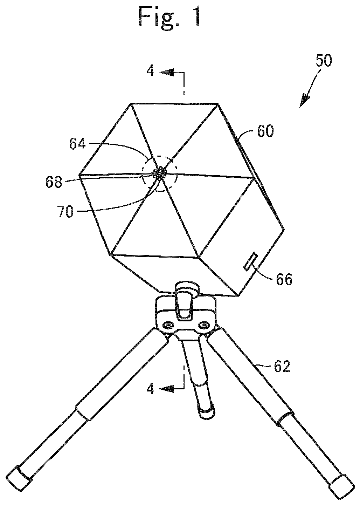

Fig. 1 shows an appearance of an aroma display 50 in accordance with a first embodiment of the present invention. The aroma display 50 has a housing 60 that is a substantially hexagonal column. At a central portion of an upper panel, which corresponds to a bottom plate of the hexagonal column of the housing 60, there is an opening section 64. At the center of the opening section 64, an opening 68 is formed, from which scent-free air from an air emission mechanism placed in the housing 60 is emitted. Around this opening, six openings including an opening 70 are formed, from which scents are emitted from six aroma cartridges (not shown in Fig. 1) loaded in the housing 60.

The aroma display 50 can be mounted on a tripod 62 for a camera. By mounting on the tripod 62, it becomes possible to place the aroma display 50 by the side of a PC, for example, with the opening section 64 of the aroma display 50 positioned very close to and directed to the user's face. This is effective to send desired scents to the user.

A micro USB connector 66 is provided on a side surface of the housing 60. Connector 66 is for feeding power to the aroma display 50 through a USB cable and to charge a built-in battery of the aroma display 50. Note that the circuitry of the aroma display 50 does not communicate with the outside through the USB cable. The circuitry inside the aroma display 50 exchanges data with the outside through wireless communication. Naturally, the inner circuitry of the aroma display 50 may perform data communication with the outside through wired communication by the USB cable.

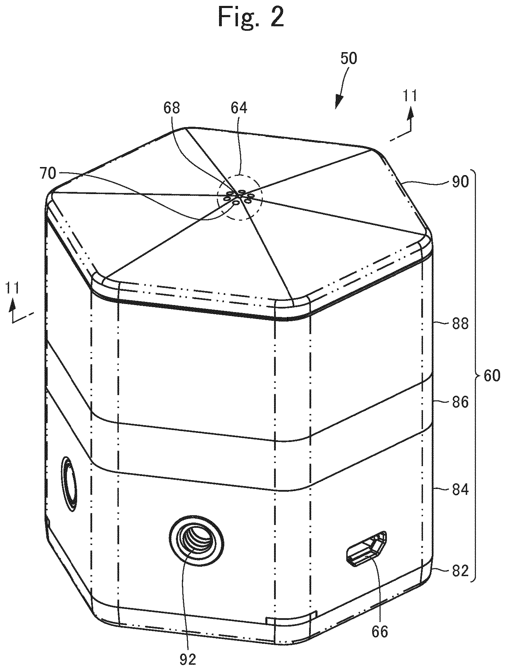

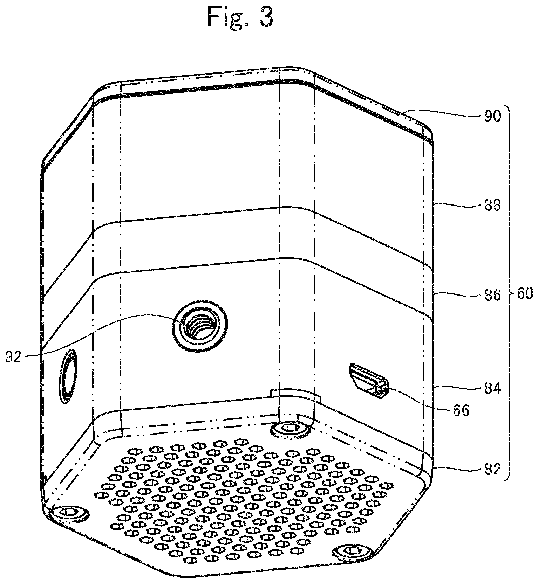

Referring to Figs. 2 to 4, the housing 60 includes: a base panel 82; a base body 84 arranged above the base panel 82 and screwed to the base panel 82; a middle body 86 fitted to the base body 84 from above; an upper body 88 fitted above the middle body 86, having a cartridge loading section opening upward, to which aroma cartridges, not shown, are loaded; and an upper panel 90 fitted to the upper body 88 to close the upper opening of the upper body 88. By removing the upper panel 90, the opening of the upper body 88 is exposed, allowing a user access to the aroma cartridge loading section, to load or remove an aroma cartridge to and from the loading section.

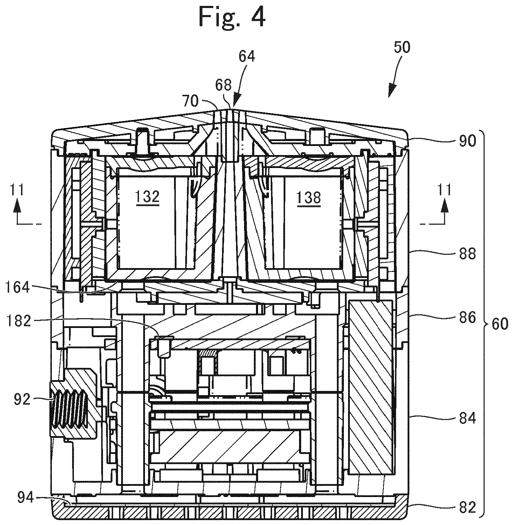

In the present embodiment, the upper body 88 has a hexagonal cross-section, and the cartridge loading section to which six aroma cartridges are loadable is formed inside the the upper body 88. As can be seen from Fig. 4, in the upper body 88, aroma cartridges 132, 138 and so on are loaded, as will be described later. On a bottom surface of the aroma cartridge 132, for example, an NFC tag 164 is attached, and at a nearby portion of the upper body 88, an NFC chip capable of wireless communication with the NFC tag 164 is provided. These will be described later.

On a side surface of the base body 84, a screw hole 92 is formed as shown in Figs. 2 to 4, to which the tripod 62 (Fig. 1) may be screwed in. The screw hole 92 is formed to meet a specific standard so that a tripod for a camera can be fitted.

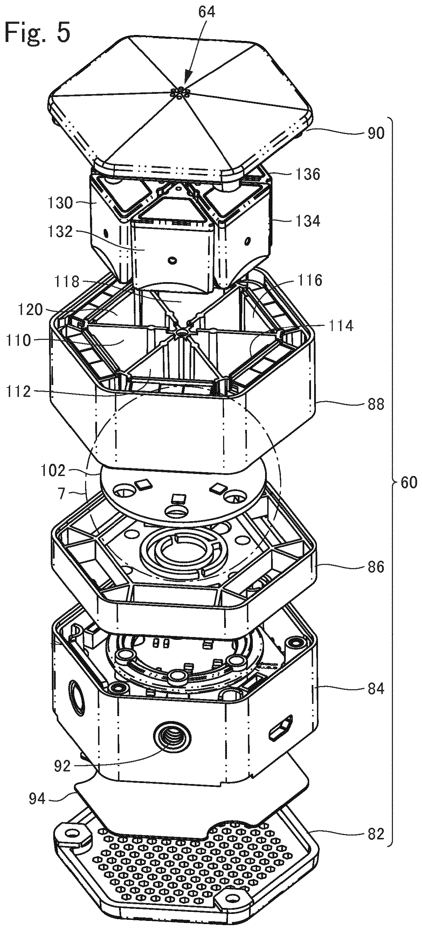

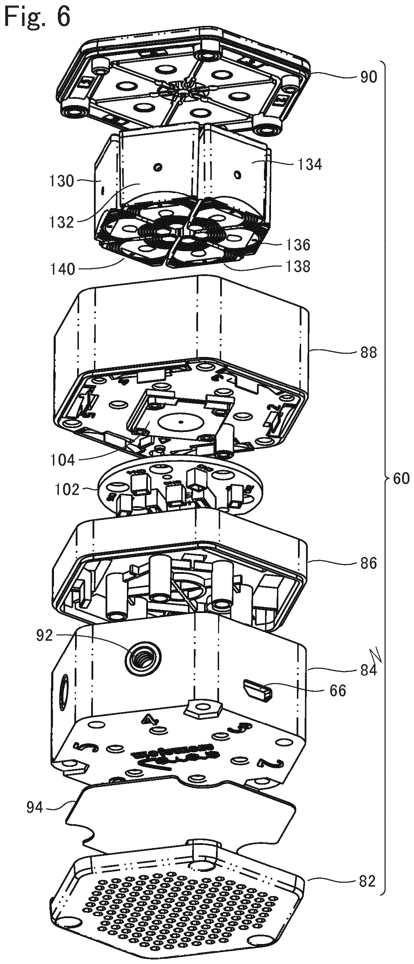

Referring to Figs. 5 and 6, the base body 84 contains: a control board having mounted thereon a communication device for wireless communication to/from the outside and a control circuitry for driving a micro blower, which will be described later; and a battery as the power source thereof. Between the middle body 86 and the upper body 88, an NFC chip stage 102 is provided. Further, on the base panel 82, a large number of small holes are formed to take in air, used to generate power to blow out scented air from the aroma cartridges. A filter 94 is provided between the base panel 82 and the base body 84 to remove dust from the introduced air.

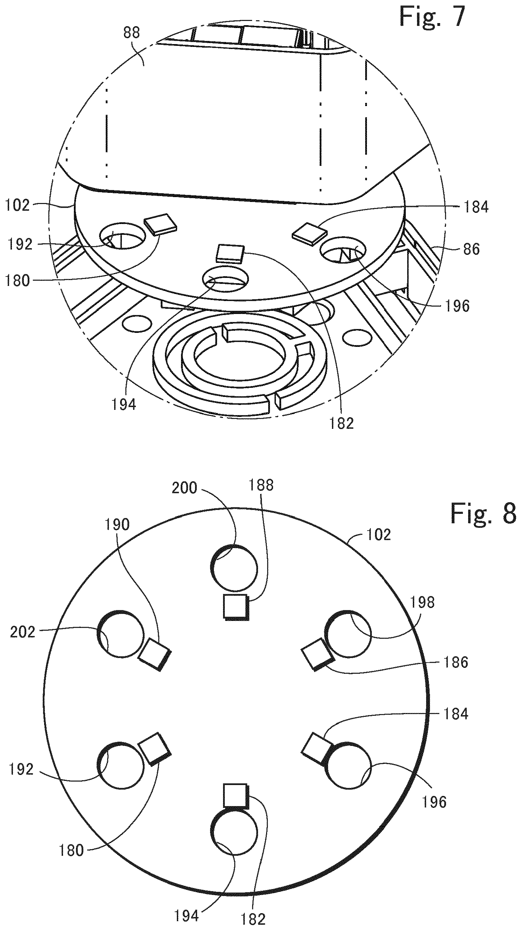

Referring to Figs. 7 and 8, on an upper surface of the NFC chip stage 102, six NFC chips 180, 182, 184, 186, 188 and 190 are fixed at positions corresponding to the aroma cartridges to be loaded. These NFC chips are NFC readers and fixed at prescribed positions with equal intervals on a circle having the center at the center of the cartridge loading section. The NFC chip stage 102 is arranged such that when the aroma cartridge is loaded to the aroma display, the upper surface of the chip stage is positioned slightly apart from the lower surface of the aroma cartridge to the side of the base body 84. When an aroma cartridge is loaded at the portion corresponding to each NFC chip, an identifier indicating the scent of the aroma cartridge can be received by NFC communication from the NFC tag attached to the lower surface of the loaded cartridge. For this reason, it is desirable that the NFC chip is arranged not close to the vertex on the edge of lower surface of the corresponding aroma cartridge but close to the opposing side where the NFC tag is attached. Openings 192, 194, 196, 198, 200 and 202 as air flow passages are formed in the vicinity of respective NFC chips.

These NFC chips 180, 182, ..., 188, 190 are respectively connected to the wireless communication device contained in the base body 84. The wireless communication device identifies the position of each NFC chip in accordance with the inputs to which the NFC chips are connected, and transmits data indicating loading positions of the aroma cartridges to the outside, together with the respective codes of the aroma cartridges received from the corresponding NFC chip. In Fig. 6, numbers (1 to 6) shown on the lower surface of the base body 84 are identifiers indicating respective loading positions and corresponding NFC chips.

Specifically referring to Fig. 6, at that portion on a lower surface of the upper body 88 which is above the NFC chip stage 102, a micro blower 104 as an air emission mechanism is fixed. The micro blower 104 and the mechanisms of the micro blowers for feeding air into each aroma cartridge will be described later.

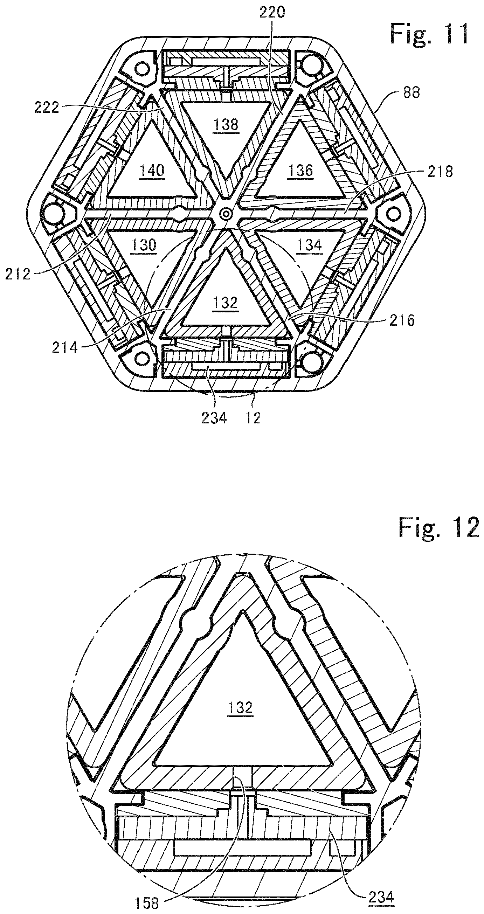

Referring to Fig. 5, the upper body 88 has a substantially hexagonal cross-sectional shape as described above. While the details will be described later with reference to Figs. 11 and 12, the space inside the upper body 88 is divided into six cartridge loading sections 110, 112, ..., 118, 120 each being a space having a cross-section of regular triangle. The aroma cartridges 130, 132, ..., 138, 140 are loaded to the respective cartridge loading sections 110, 112, ..., 118, 120.

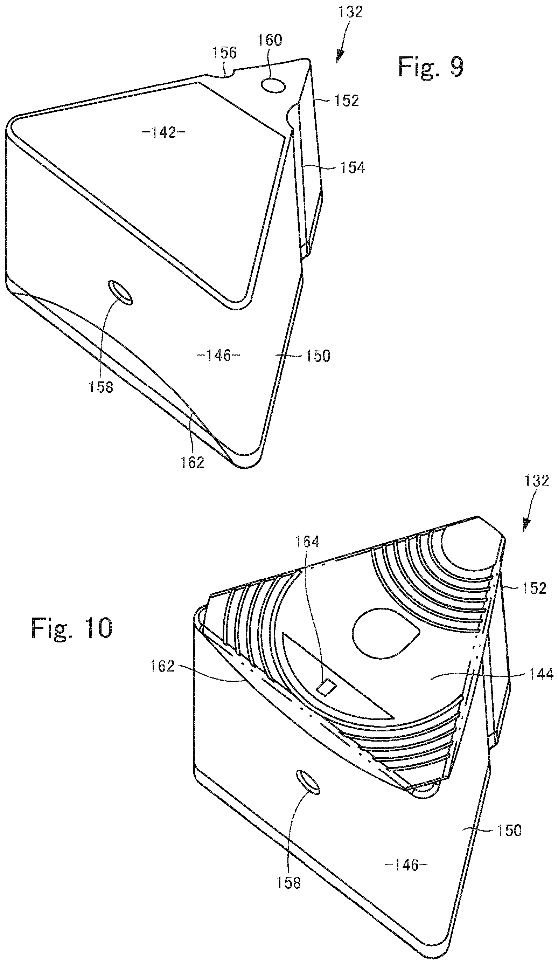

Referring to Figs. 9 and 10, by way of example, the aroma cartridge 132 has a housing 150 of which cross-section is substantially a regular triangle, consisting of upper and lower surfaces 142 and 144 parallel to and congruent with each other, and a side surface 146 formed to connect their circumferences. The housing 150 has one edge 152 in the longitudinal direction, and on two side surfaces on both sides of the edge 152, grooves 154 and 156 are formed near the edge 152.

Referring to Fig. 9, in an area of the upper surface 142 surrounded by the edge 152 and the grooves 154 and 156, an opening 160 is formed, which is connected to a hollow space inside the housing 150 for emitting scented air from the scent source sealed in the housing 150. On that portion of the side surface 146 opposite to the edge 152, is an opening 158 formed for feeding air from an outside micro blower into the housing 150.

Referring to Fig. 10, on the lower surface 144 of the housing 150, an NFC tag 164 is attached by means of a sticker 162, for transmitting the identifier of an aroma cartridge to the aroma display 50 by the near field communication with an NFC chip. In the present embodiment, the sticker 162 extends from the lower surface 144 and partly reaches the side surface 146. The NFC tag 164 on the sticker 162 is commercially available and it is used in the present embodiment. The sticker 162 can be peeled off from the housing 150 and, therefore, it is possible to remove the NFC tag 164 from the housing 150 and replace it with another as needed. The NFC tag 164 is attached to a lower surface 44 on a position near the opposite side of the edge 152. By attaching the NFC tag 164 at such a position, the distance between the NFC tags of adjacent aroma cartridges can be made longer when a plurality of aroma cartridges are loaded to the aroma display 50. As a result, the distance from the NFC tag to its corresponding NFC chip becomes shorter than the distance to other NFC chips, ensuring correct communication.

Referring to Fig. 11, the upper body 88 has the shape of a substantially hexagonal column as described above. Its inside is divided by division walls 212, 214, ..., 220, 222 extending from the center of the hexagon to each vertex, into the aroma cartridge loading sections each having a cross-section of substantially a regular triangle. At portions of the division walls 212, ..., 222 close to the center of the hexagon, protruding portions that engage with the grooves 154 and 156 shown in Fig. 9 are formed extending upward. Therefore, when an aroma cartridge is to be loaded to the cartridge loading section, it can be correctly positioned by positioning the grooves 154 and 156 to be engaged to the protruding portions of the division wall.

When each cartridge is loaded to the cartridge loading section, it follows that the opening for introducing air into the aroma cartridge faces outward from the upper body 88. For example, referring to Fig. 12, the aroma cartridge 132 has its opening 158 facing outward. Between the upper body 88 and the aroma cartridge 132, a micro blower 234 is arranged for feeding air into the opening 158. By driving the micro blower 234, air is fed into the aroma cartridge 132, and scented air is emitted to the outside from the opening 160 (shown in Fig. 9) positioned near the center for scent emission of the aroma cartridge 132.

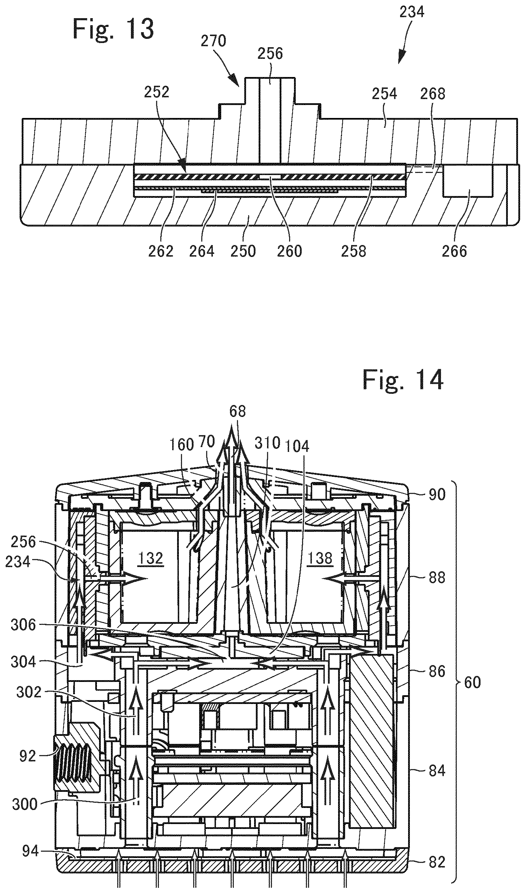

Micro blower 234 has the same structure as disclosed in JP2014-092673A mentioned above. Referring to Fig. 13, specifically, the micro blower 234 has a case 250 with a blower chamber 252 as an inner space, and a cover member 254 with a nozzle 270 formed on its upper surface, attached to the upper surface of case 250 so that it covers the upper surface of case 250 to close the blower chamber 252. An air channel 256 is formed in nozzle 270, and air channel 256 opens into the upper portion of blower chamber 252.

Blower chamber 252 is divided into upper and lower two portions by a partition plate 258. At a position immediately below air channel 256 of partition plate 258, an opening 260 is formed aligned with air channel 256.

In the lower space of blower chamber 252 divided by the partition plate 258, a diaphragm 262 formed of a thin metal plate with spring resiliency and a piezoelectric element 264 bonded to the lower surface of diaphragm 262 are provided. By applying AC voltage to piezoelectric element 264, diaphragm 262 vibrates up and down and emits air through opening 260 to blower chamber 252 and further through air channel 256 to the outside. By positioning the micro blower 234 inside the upper body 88 such that nozzle 270 is positioned at the opening on the back side of the aroma cartridge, air can be introduced into the aroma cartridge at a desired timing for a desired time period, and thus, scented air can be emitted from the aroma cartridge. It is noted that an air intake opening 266 is formed in case 250, and the air from intake opening 266 is fed through an air channel 268 to blower chamber 252.

Each of the aroma cartridges other than the aroma cartridge 132 also has a micro blower arranged for feeding air into the aroma cartridge through the opening on the back side. By operating these independently from each other, it is possible to emit the scented air at a desired timing for a desired time period from a desired aroma cartridge. The micro blower 104 shown in Fig. 6 also has the same structure as this one and it can emit air at a desired timing from the opening 68 shown in Figs. 1, 2, and 4.

Fig. 14 shows the air flow inside the aroma display 50. Description will be given with reference to the aroma cartridge 132. The base panel 82 has many openings through which air is taken into the housing 60. The air passes through air flow paths 300 and 302 formed in the base body 84 and the middle body 86, part of the air passes through air flow path 306 to the micro blower 104 and the remaining part passes through air flow path 304 formed in the upper body 88 to the micro blower 234. When AC voltage is applied to the micro blower 234, the air emitted from the micro blower 234 passes through the air channel 256 into the aroma cartridge 132.

When air is introduced by the micro blower 234 to the aroma cartridge 132, scented air inside the aroma cartridge 132 is emitted to the outside through a path from the opening 160 of the aroma cartridge 132 to the opening 70 of the upper panel 90. The flow is the same in other aroma cartridges.

On the other hand, when AC voltage is applied to the micro blower 104, the air fed into the micro blower 104 passes through an air flow path 310 and emitted to the outside through the opening 68 formed in the upper panel 90. When scented air is emitted from an aroma cartridge and thereafter air is emitted through the opening 68, the lingering scent is dissipated and effective switch to another scent becomes possible.

The air emitted from the opening 68 by driving the micro blower 104 by AC voltage also has a boosting function that carries the scented air emitted from the opening 70 farther away, in addition to the function of dissipating the lingering air. Micro blower 104 may have the same size as micro blower 234 for feeding air to the aroma cartridge 132. For this purpose, however, it is preferable to have a larger size and a structure capable of feeding more air with more strength than micro blower 234. Further, by adjusting the amount of air emitted from the opening 68 by the micro blower 104, it is possible to adjust the density of the scent emitted from the opening 70. Further, by adjusting the amount of air from the openings 68 and 70, it is possible to implement a perfume blending function of the aroma display 50.

### <Operation>

Aroma display 50 described above operates in the following manner.

As a premise, it is assumed that aroma cartridges and the aroma display 50 are sold separately. Each aroma cartridge has a label indicating the scent source contained therein, and an NFC tag storing an identifier allocated beforehand to the scent source is attached on the bottom surface. A user of the aroma display 50 buys an aroma cartridge or aroma cartridges he/she likes and loads them to the aroma display 50 in the following manner.

Referring to Fig. 5, by removing the upper panel 90 from the housing 60, the upper opening of the upper body 88 is exposed. The user loads each of the aroma cartridges to any cartridge loading section therein. For example, he/she may load the aroma cartridges to all six cartridge loading sections. Thereafter, the upper panel 90 is attached to the housing 60.

Referring to Fig. 4, when aroma cartridges are loaded to the upper body 88, the NFC tag 164 attached to each aroma cartridge will be positioned near the corresponding NFC chip of the loading section where the aroma cartridge is loaded, among the NFC chips 180, 182, ..., 188, 190 provided on the NFC chip stage 102 in the middle body 86. As a result, the NFC tag 164 transmits the scent identifier of the aroma cartridge to the corresponding NFC chip by near field communication with the NFC chip. It is particularly noted that the NFC tag will be positioned near the outer periphery of the bottom surface of aroma cartridge, and the distance between the NFC tags of adjacent aroma cartridges is long. As a result, each NFC chip communicates correctly with the NFC tag of the corresponding aroma cartridge, and communication error can be prevented.

In the present embodiment, the scent identifier is transmitted to the wireless communication device. The wireless communication device transmits information specifying the NFC chip that has transmitted the scent identifier (information specifying the position of loading section corresponding to the NFC chip) and its associated scent identifier to an external PC, for example, for controlling the entire operation of the aroma display 50. Then, it becomes possible for the external PC to know which aroma cartridge is loaded to which cartridge loading section of the aroma display 50.

Assume that there is a scenario for a movie reproduced on a PC, for example, to emit a specific scent identified by the identifier at a specific scene for a specific time period in synchronization with the movie. Then the PC reads the scenario and reproduces the scenario to be synchronized with the movie. Specifically, the PC transmits a command to the wireless communication device of the aroma display 50 such that a specific scent is emitted from the aroma cartridge with the designated identifier at the timing designated by the scenario. Receiving the command, the wireless communication device applies AC voltage at the designated timing for the designated time period to the micro blower of the cartridge loading section on which the designated scent is loaded. As a result, the designated scent is emitted from the aroma display 50 at the timing and for the duration as designated by the scenario.

As described above, in order to reproduce various scents by the aroma display 50, it is necessary for the PC to know beforehand which aroma cartridge with specific identifier is loaded at which loading section of the aroma display 50. For this purpose, conventionally, it has been necessary to manually specify which cartridges are loaded at which positions. When one uses the aroma display 50 in accordance with the above-described embodiment, what is necessary is to simply select which cartridges (which scents) are to be loaded in the aroma display 50. There is no need to care about where to load which aroma cartridges. Further, it is unnecessary to manually input which cartridge is loaded at which position to a device controlling the aroma display 50. Therefore, by the aroma cartridges and the aroma display provided by the present invention, reliable control of aroma cartridges becomes possible, and the possibility of emitting the scent that is not intended can be reduced.

In the embodiment above, an NFC tag is attached to the bottom surface of each aroma cartridge. The present invention, however, is not limited to such an embodiment. An NFC tag may be attached to a side surface of each aroma cartridge and an NFC chip may be arranged on the division wall of an aroma cartridge loading section of the aroma display. Further, if it is possible to attach an NFC chip on the rear surface of the upper panel 90, an NFC tag may be attached on the upper surface of an aroma cartridge. An NFC tag may be embedded inside rather than attached to the outer surface of each aroma cartridge. Alternatively, a slot may be formed in the housing of an aroma cartridge, and an NFC tag may be fitted therein.

Further, in the embodiment above, the aroma cartridge is loaded to the cartridge loading section from above by removing the upper panel 90 of the housing 60. The present invention, however, is not limited to such an embodiment. By way of example, a door may be provided on a side surface of the housing of the cartridge loading section, and the cartridge may be loaded from the side surface. In this case also, the positions of the NFC tag and the NFC chip may be appropriately determined to enable communication between the two.

Further, in the embodiment above, a scent identifier is stored in the NFC tag of each aroma cartridge. The present invention, however, is not limited to such an embodiment. Assume, for example, that aroma cartridges manufactured by different companies or aroma cartridges imported from abroad are to be used. The scent identifiers of these cartridges may or may not be common and if not, the scent type cannot be specified by the identifier only. In that case, another identifier indicating the origin such as the country or manufacturer may be necessary. A service translating identifiers indicating different countries or different manufacturers and identifiers used for scents in different countries and by different manufacturers to common identifiers would be helpful, since by accessing such a service, an identifier of any scent can be translated to a common identifier and hence wider variety of aroma cartridges become available.

Alternatively, each aroma cartridge may have its own identifier that is stored in an NFC tag, and information of identifiers of respective aroma cartridges combined with information specifying corresponding scents may be posted on a network to allow searching. By reading a cartridge identifier from its NFC tag and by specifying the scent corresponding to the identifier on the network, the same effects as described in the embodiment above can be attained.

In the embodiment above, the number of cartridges that can be used at one time is six. The present invention, however, is not limited to such an embodiment. Provided that the opening for emitting scents can be formed in a very limited area, a larger number of aroma cartridges can be used. In that case, the trouble of inputting larger number of identifiers can significantly be reduced, and use of the aroma display becomes more convenient. In the embodiment above, an example has been described in which scent is emitted from a single aroma cartridge. The present invention, however, is not limited to such an embodiment. By emitting a plurality of different scents at one time, a scent very much different from any of the scents of any single aroma cartridges loaded in the aroma display can be produced.

The embodiments as have been described here are mere examples and should not be interpreted as restrictive. The scope of the present invention is determined by each of the claims with appropriate consideration of the written description of the embodiments and embraces modifications within the meaning of, and equivalent to, the languages in the claims.

## Claims

1. An aroma cartridge, comprising
a columnar housing having first and second surfaces each substantially having a shape of a triangle parallel to and congruent with each other and a side surface connecting the first and second surfaces; wherein
said housing has a hollow portion to store a scent source, a scent passage opening at said first surface for emitting scent from the scent source in the hollow portion to the outside, connecting said hollow portion to the outside, and an opening for introducing air from outside to said hollow portion;
each of said first and second surfaces has a corresponding first vertex;
said scent passage is opened at a portion near said first vertex of said first surface;
said aroma cartridge further comprising
a near field communication tag provided on said second surface or said side surface of said housing for transmitting identification data for identifying type of scent source contained in the aroma cartridge to a near field communication chip by near field communication; wherein
said near field communication tag is arranged on the surface of said housing, at a position closer to said second surface than said first surface and closer to an opposite side of said first vertex of said second surface than said first vertex of said second surface,
said opening is formed on said side surface, and
said near field communication tag is arranged at a position closer to the center of said opposite side than said first vertex on said second surface of said housing.

2. The aroma cartridge according to claim 1, wherein said triangle is an isosceles triangle and said first vertex is the vertex formed by two sides of equal length of said isosceles triangle.

3. The aroma cartridge according to claim 2, wherein said triangle is an equilateral triangle.

4. The aroma cartridge according to claim 3, wherein said near field communication tag is attached to said housing by an adhesive sticker.

5. The aroma cartridge according to claim 1, wherein said near field communication tag is arranged at a position closer to the center of said opposite side than said first vertex on said second surface of said housing.

6. The aroma cartridge according to claim 1, wherein said triangle is an isosceles triangle and said first vertex is the vertex formed by two sides of equal length of said isosceles triangle.

7. The aroma cartridge according to claim 1, wherein said triangle is an equilateral triangle.

8. The aroma cartridge according to claim 1, wherein said near field communication tag is attached to said housing by an adhesive sticker.

9. An aroma display having a cartridge loading section for containing a plurality of aroma cartridges, for emitting scent from each of said plurality of aroma cartridges; wherein
each of said plurality of aroma cartridges includes
a columnar housing having first and second surfaces each substantially having a shape of a triangle parallel to and congruent with each other and a side surface connecting the first and second surfaces;
said housing has a hollow portion to store a scent source, a scent passage opening at said first surface for emitting scent from the scent source in the hollow portion to the outside, connecting said hollow portion to the outside, and an opening for introducing air from outside to said hollow portion;
each of said first and second surfaces has a corresponding first vertex;
said scent passage is opened at a portion near said first vertex of said first surface;
said aroma cartridge further includes
a near field communication tag provided on said second surface or said side surface of said housing for transmitting identification data for identifying type of scent source contained in the aroma cartridge to a near field communication chip by near field communication;
said near field communication tag is arranged on the surface of said housing, at a position closer to said second surface than said first surface and closer to an opposite side of said first vertex of said second surface than said first vertex of said second surface; wherein
said opening is formed on said side surface, and
said near field communication tag is arranged at a position closer to the center of said opposite side than said first vertex on said second surface of said housing;
said cartridge loading section of said aroma display has an inner shape rotationally symmetric about a prescribed center, and receives each said aroma cartridge such that said first vertex is positioned adjacent to said center and such that said second surfaces of said plurality of aroma cartridges are aligned on one plane;
said aroma display further comprising
a chip stage mounting a plurality of near field communication chips arranged at positions enabling communication with said near field communication tag of any of said plurality of aroma cartridges, respectively corresponding to said plurality of aroma cartridges loaded in said cartridge loading section, wherein
each of said plurality of near field communication chips is arranged at a position closer to the center of the opposite side of said first vertex than said first vertex of said second surface of one of said plurality of aroma cartridges.

10. The aroma display according to claim 9, wherein each of said near field communication chips receives, when said aroma cartridge is loaded at a portion close to said near field communication chip, data identifying scent source contained in said aroma cartridge by near field communication with said near field communication tag of said aroma cartridge, and outputs the data identifying the scent source and data identifying said near field communication tag;
said aroma display further comprising a control circuit receiving said data identifying the scent source from each of said near field communication chips, and based on the data, driving that aroma cartridge which has a desired scent source to emit the scent.

11. The aroma display according to claim 10, wherein said plurality of near field communication chips are arranged on a circle centered at said prescribed center.

12. The aroma display according to claim 11, wherein said plurality of near field communication chips are arranged on said circle at an equal distance from each other.

13. The aroma display according to claim 12 wherein said circle is on the side opposite to said first surface with respect to said plane on which said second surfaces are aligned..

14. An aroma display system comprising one or a plurality of aroma cartridges and an aroma display emitting scent from the plurality of aroma cartridges; wherein
each of said plurality of aroma cartridges includes
a columnar housing having first and second surfaces each substantially having a shape of a triangle parallel to and congruent with each other and a side surface connecting the first and second surfaces;
said housing has a hollow portion to store a scent source, a scent passage opening at said first surface for emitting scent from the scent source in the hollow portion to the outside, connecting said hollow portion to the outside, and an opening for introducing air from outside to said hollow portion;
each of said first and second surfaces has a corresponding first vertex;
said scent passage is opened at a portion near said first vertex of said first surface;
said aroma cartridge further includes
a near field communication tag provided on said second surface or said side surface of said housing for transmitting identification data for identifying type of scent source contained in the aroma cartridge to a near field communication chip by near field communication;
said near field communication tag is arranged on the surface of said housing, at a position closer to said second surface than said first surface and closer to an opposite side of said first vertex of said second surface than said first vertex of said second surface; wherein
said opening is formed on said side surface, and
said near field communication tag is arranged at a position closer to the center of said opposite side than said first vertex on said second surface of said housing;
said aroma display has a cartridge loading section for containing a plurality of aroma cartridges;
said cartridge loading section has an inner shape rotationally symmetric about a prescribed center, and receives each said aroma cartridge such that said first vertex is positioned adjacent to said center and such that said second surfaces of said plurality of aroma cartridges are aligned on one plane;
said aroma display further includes a chip stage mounting a plurality of near field communication chips arranged at positions enabling communication with said near field communication tag of any of said plurality of aroma cartridges, respectively corresponding to said plurality of aroma cartridges loaded in said cartridge loading section, and wherein
each of said plurality of near field communication chips is arranged at a position closer to the center of the opposite side of said first vertex than said first vertex of said second surface of one of said plurality of aroma cartridges.

## Patentansprüche

1. Duftpatrone, umfassend
ein säulenförmiges Gehäuse mit einer ersten und einer zweiten Oberfläche, die jeweils im Wesentlichen eine Form eines Dreiecks aufweisen und zueinander parallel und kongruent sind, und einer Seitenoberfläche, die die erste und die zweite Oberfläche verbindet, wobei
das Gehäuse Folgendes aufweist: einen hohlen Abschnitt zum Aufbewahren einer Duftstoffquelle, einen an der ersten Oberfläche öffnenden Duftstoffkanal zum Ausstoßen von Duftstoff von der Duftstoffquelle in dem hohlen Abschnitt nach außen, der den hohlen Abschnitt mit außen verbindet, und eine Öffnung zum Einbringen von Luft von außen in den hohlen Abschnitt;
die erste und die zweite Oberfläche jeweils einen entsprechenden ersten Eckpunkt aufweisen;
der Duftstoffkanal an einem Abschnitt in der Nähe des ersten Eckpunkts der ersten Oberfläche geöffnet ist;
wobei die Duftpatrone ferner Folgendes umfasst:
einen auf der zweiten Oberfläche oder der Seitenoberfläche des Gehäuses bereitgestellten Nahfeldkommunikations-Tag zum Übertragen von Identifikationsdaten zum Identifizieren der Art der in der Duftpatrone enthaltenen Duftstoffquelle an einen Nahfeldkommunikations-Chip durch Nahfeldkommunikation; wobei
der Nahfeldkommunikations-Tag an einer Stelle näher an der zweiten Oberfläche als der ersten Oberfläche und näher an einer Gegenseite des ersten Eckpunkts der zweiten Oberfläche als dem ersten Eckpunkt der zweiten Oberfläche auf der Oberfläche des Gehäuses angeordnet ist,
die Öffnung auf der Seitenoberfläche gebildet ist und
der Nahfeldkommunikations-Tag an einer Stelle näher an dem Mittelpunkt der Gegenseite als dem ersten Eckpunkt auf der zweiten Oberfläche des Gehäuses angeordnet ist.

2. Duftpatrone nach Anspruch 1, wobei das Dreieck ein gleichschenkliges Dreieck ist und der erste Eckpunkt der von zwei Seiten gleicher Länge des gleichschenkligen Dreiecks gebildete Eckpunkt ist.

3. Duftpatrone nach Anspruch 2, wobei das Dreieck ein gleichseitiges Dreieck ist.

4. Duftpatrone nach Anspruch 3, wobei der Nahfeldkommunikations-Tag durch ein Klebeetikett an dem Gehäuse angebracht ist.

5. Duftpatrone nach Anspruch 1, wobei der Nahfeldkommunikations-Tag an einer Stelle näher an dem Mittelpunkt der Gegenseite als dem ersten Eckpunkt auf der zweiten Oberfläche des Gehäuses angeordnet ist.

6. Duftpatrone nach Anspruch 1, wobei das Dreieck ein gleichschenkliges Dreieck ist und der erste Eckpunkt der von zwei Seiten gleicher Länge des gleichschenkligen Dreiecks gebildete Eckpunkt ist.

7. Duftpatrone nach Anspruch 1, wobei das Dreieck ein gleichseitiges Dreieck ist.

8. Duftpatrone nach Anspruch 1, wobei der Nahfeldkommunikations-Tag durch ein Klebeetikett an dem Gehäuse angebracht ist.

9. Duftdisplay mit einem Partonenladeabschnitt zum Enthalten einer Vielzahl von Duftpatronen zum Ausstoßen von Duftstoff aus jeder der Vielzahl von Duftpatronen; wobei
jede der Vielzahl von Duftpatronen Folgendes umfasst:
ein säulenförmiges Gehäuse mit einer ersten und einer zweiten Oberfläche, die jeweils im Wesentlichen eine Form eines Dreiecks aufweisen und zueinander parallel und kongruent sind, und einer Seitenoberfläche, die die erste und die zweite Oberfläche verbindet;
das Gehäuse Folgendes aufweist: einen hohlen Abschnitt zum Aufbewahren einer Duftstoffquelle, einen an der ersten Oberfläche öffnenden Duftstoffkanal zum Ausstoßen von Duftstoff von der Duftstoffquelle in dem hohlen Abschnitt nach außen, der den hohlen Abschnitt mit außen verbindet, und eine Öffnung zum Einbringen von Luft von außen in den hohlen Abschnitt;
die erste und die zweite Oberfläche jeweils einen entsprechenden ersten Eckpunkt aufweisen;
der Duftstoffkanal an einem Abschnitt in der Nähe des ersten Eckpunkts der ersten Oberfläche geöffnet ist;
wobei Duftpatrone ferner Folgendes umfasst:
einen auf der zweiten Oberfläche oder der Seitenoberfläche des Gehäuses bereitgestellten Nahfeldkommunikations-Tag zum Übertragen von Identifikationsdaten zum Identifizieren der Art der in der Duftpatrone enthaltenen Duftstoffquelle an einen Nahfeldkommunikations-Chip durch Nahfeldkommunikation;
der Nahfeldkommunikations-Tag an einer Stelle näher an der zweiten Oberfläche als der ersten Oberfläche und näher an einer Gegenseite des ersten Eckpunkts der zweiten Oberfläche als dem ersten Eckpunkt der zweiten Oberfläche auf der Oberfläche des Gehäuses angeordnet ist; wobei
die Öffnung auf der Seitenoberfläche gebildet ist und
der Nahfeldkommunikations-Tag an einer Stelle näher an dem Mittelpunkt der Gegenseite als dem ersten Eckpunkt auf der zweiten Oberfläche des Gehäuses angeordnet ist;
der Partonenladeabschnitt des Duftdisplays eine um einen vorgegebenen Mittelpunkt rotationssymmetrische innere Form aufweist und jede Duftpatrone derart, dass der erste Eckpunkt dem Mittelpunkt benachbart positioniert ist, und derart, dass die zweiten Oberflächen der Vielzahl von Duftpatronen auf einer Ebene ausgerichtet sind, aufnimmt;
wobei das Duftdisplay ferner Folgendes umfasst:
einen Chiphalter, der eine Vielzahl von Nahfeldkommunikations-Chips hält, die an Stellen angeordnet sind, die die Kommunikation mit dem Nahfeldkommunikations-Tag jeglicher der Vielzahl von Duftpatronen ermöglichen, die jeweils einer der Vielzahl von in den Patronenladeabschnitt geladenen Duftpatronen entsprechen, wobei
jeder der Vielzahl von Nahfeldkommunikations-Chips an einer Stelle näher an dem Mittelpunkt der Gegenseite des ersten Eckpunkts als dem ersten Eckpunkt der zweiten Oberfläche einer der Vielzahl von Duftpatronen angeordnet ist.

10. Duftdisplay nach Anspruch 9, wobei jeder der Nahfeldkommunikations-Chips, wenn die Duftpatrone an einem Abschnitt nah an dem Nahfeldkommunikations-Chip geladen ist, die Daten, die die in der Duftpatrone enthaltene Duftstoffquelle identifizieren, durch Nahfeldkommunikation mit dem Nahfeldkommunikation-Tag der Duftpatrone empfängt und die Daten, die die Duftstoffquelle identifizieren, und Daten, die den Nahfeldkommunikations-Tag identifizieren, ausgibt;
wobei das Duftdisplay ferner eine Steuerschaltung umfasst, die die Daten empfängt, die die Duftstoffquelle von jedem der Nahfeldkommunikations-Chips identifizieren, und basierend auf den Daten diejenige Duftpatrone ansteuert, die eine gewünschte Duftstoffquelle aufweist, um den Duftstoff auszustoßen.

11. Duftdisplay nach Anspruch 10, wobei die Vielzahl von Nahfeldkommunikations-Chips auf einem an dem vorgegebenen Mittelpunkt zentrierten Kreis angeordnet sind.

12. Duftdisplay nach Anspruch 11, wobei die Vielzahl von Nahfeldkommunikations-Chips in gleichen Abständen voneinander auf dem Kreis angeordnet sind.

13. Duftdisplay nach Anspruch 12, wobei der Kreis bezogen auf die Ebene, auf der die zweiten Oberflächen ausgerichtet sind, auf der der ersten Oberfläche gegenüberliegenden Seite liegt.

14. Duftdisplaysystem, umfassend eine oder eine Vielzahl von Duftpatronen und ein Duftdisplay, das Duftstoff aus der Vielzahl von Duftpatronen ausstößt, wobei
jede der Vielzahl von Duftpatronen Folgendes umfasst:
ein säulenförmiges Gehäuse mit einer ersten und einer zweiten Oberfläche, die jeweils im Wesentlichen eine Form eines Dreiecks aufweisen und zueinander parallel und kongruent sind, und einer Seitenoberfläche, die die erste und die zweite Oberfläche verbindet;
das Gehäuse Folgendes aufweist: einen hohlen Abschnitt zum Aufbewahren einer Duftstoffquelle, einen an der ersten Oberfläche öffnenden Duftstoffkanal zum Ausstoßen von Duftstoff von der Duftstoffquelle in dem hohlen Abschnitt nach außen, der den hohlen Abschnitt mit außen verbindet, und eine Öffnung zum Einbringen von Luft von außen in den hohlen Abschnitt;
die erste und die zweite Oberfläche jeweils einen entsprechenden ersten Eckpunkt aufweisen;
der Duftstoffkanal an einem Abschnitt in der Nähe des ersten Eckpunkts der ersten Oberfläche geöffnet ist;
wobei die Duftpatrone ferner Folgendes umfasst:
einen auf der zweiten Oberfläche oder der Seitenoberfläche des Gehäuses bereitgestellten Nahfeldkommunikations-Tag zum Übertragen von Identifikationsdaten zum Identifizieren der Art der in der Duftpatrone enthaltenen Duftstoffquelle an einen Nahfeldkommunikations-Chip durch Nahfeldkommunikation;
der Nahfeldkommunikations-Tag an einer Stelle näher an der zweiten Oberfläche als der ersten Oberfläche und näher an einer Gegenseite des ersten Eckpunkts der zweiten Oberfläche als dem ersten Eckpunkt der zweiten Oberfläche auf der Oberfläche des Gehäuses angeordnet ist; wobei
die Öffnung auf der Seitenoberfläche gebildet ist und
der Nahfeldkommunikations-Tag an einer Stelle näher an dem Mittelpunkt der Gegenseite als dem ersten Eckpunkt auf der zweiten Oberfläche des Gehäuses angeordnet ist;
das Duftdisplay einen Patronenladeabschnitt zum Enthalten einer Vielzahl von Duftpatronen aufweist;
der Partonenladeabschnitt eine um einen vorgegebenen Mittelpunkt rotationssymmetrische innere Form aufweist und jede Duftpatrone derart aufnimmt, dass der erste Eckpunkt dem Mittelpunkt benachbart positioniert ist, und dass die zweiten Oberflächen der Vielzahl von Duftpatronen auf einer Ebene ausgerichtet sind;
das Duftdisplay ferner einen Chiphalter umfasst, der eine Vielzahl von Nahfeldkommunikations-Chips hält, die an Stellen angeordnet sind, die die Kommunikation mit dem Nahfeldkommunikations-Tag jeglicher der Vielzahl von Duftpatronen ermöglichen, die jeweils einer der Vielzahl von in den Patronenladeabschnitt geladenen Duftpatronen entsprechen, und wobei
jeder der Vielzahl von Nahfeldkommunikations-Chips an einer Stelle näher an dem Mittelpunkt der Gegenseite des ersten Eckpunkts als dem ersten Eckpunkt der zweiten Oberfläche einer der Vielzahl von Duftpatronen angeordnet ist.

## Revendications

1. Cartouche d'arôme, comportant
un logement en forme de colonne ayant des première et deuxième surfaces qui, chacune, ont sensiblement une forme de triangle et qui sont parallèles et congruentes l'une par rapport à l'autre et une surface latérale qui relie les première et deuxième surfaces ; dans laquelle
ledit logement a une partie creuse servant à stocker une source de parfum, un passage pour parfum s'ouvrant au niveau de ladite première surface servant à émettre un parfum en provenance de la source de parfum dans la partie creuse jusqu'à l'extérieur, raccordant ladite partie creuse à l'extérieur, et une ouverture servant à introduire de l'air en provenance de l'extérieur jusqu'à ladite partie creuse ;
chacune desdites première et deuxième surfaces a un premier sommet correspondant ;
ledit passage pour parfum est ouvert au niveau d'une partie proche dudit premier sommet de ladite première surface ;
ladite cartouche d'arôme comportant par ailleurs
une étiquette de communication en champ proche mise en oeuvre sur ladite deuxième surface ou sur ladite surface latérale dudit logement servant à transmettre des données d'identification pour identifier le type de la source de parfum contenue dans la cartouche d'arôme à une puce de communication en champ proche par la communication en champ proche ; dans laquelle
ladite étiquette de communication en champ proche est agencée sur la surface dudit logement, au niveau d'une position plus proche de ladite deuxième surface que de ladite première surface et plus proche d'un côté opposé dudit premier sommet de ladite deuxième surface que dudit premier sommet de ladite deuxième surface,
ladite ouverture est formée sur ladite surface latérale, et
ladite étiquette de communication en champ proche est agencée au niveau d'une position plus proche du centre dudit côté opposé que dudit premier sommet sur ladite deuxième surface dudit logement.

2. Cartouche d'arôme selon la revendication 1, dans laquelle ledit triangle est un triangle isocèle et ledit premier sommet est le sommet formé par deux côtés de longueur égale dudit triangle isocèle.

3. Cartouche d'arôme selon la revendication 2, dans laquelle ledit triangle est un triangle équilatéral.

4. Cartouche d'arôme selon la revendication 3, dans laquelle ladite étiquette de communication en champ proche est attachée audit logement par un autocollant adhésif.

5. Cartouche d'arôme selon la revendication 1, dans laquelle ladite étiquette de communication en champ proche est agencée au niveau d'une position plus proche du centre dudit côté opposé que dudit premier sommet sur ladite deuxième surface dudit logement.

6. Cartouche d'arôme selon la revendication 1, dans laquelle ledit triangle est un triangle isocèle et ledit premier sommet est le sommet formé par deux côtés de longueur égale dudit triangle isocèle.

7. Cartouche d'arôme selon la revendication 1, dans laquelle ledit triangle est un triangle équilatéral.

8. Cartouche d'arôme selon la revendication 1, dans laquelle ladite étiquette de communication en champ proche est attachée audit logement par un autocollant adhésif.

9. Dispositif de présentation d'arômes ayant une section de chargement de cartouches servant à contenir une pluralité de cartouches d'arôme, pour émettre un parfum en provenance de chacune de ladite pluralité de cartouches d'arôme ; dans lequel
chacune de ladite pluralité de cartouches d'arôme comprend
un logement en forme de colonne ayant des première et deuxième surfaces qui, chacune, ont sensiblement une forme de triangle et qui sont parallèles et congruentes l'une par rapport à l'autre et une surface latérale qui relie les première et deuxième surfaces ;
ledit logement a une partie creuse servant à stocker une source de parfum, un passage pour parfum s'ouvrant au niveau de ladite première surface servant à émettre un parfum en provenance de la source de parfum dans la partie creuse jusqu'à l'extérieur, raccordant ladite partie creuse à l'extérieur, et une ouverture servant à introduire de l'air en provenance de l'extérieur jusqu'à ladite partie creuse ;
chacune desdites première et deuxième surfaces a un premier sommet correspondant ;
ledit passage pour parfum est ouvert au niveau d'une partie proche dudit premier sommet de ladite première surface ;
ladite cartouche d'arôme comprend par ailleurs
une étiquette de communication en champ proche mise en oeuvre sur ladite deuxième surface ou sur ladite surface latérale dudit logement servant à transmettre des données d'identification pour identifier le type de la source de parfum contenue dans la cartouche d'arôme à une puce de communication en champ proche par la communication en champ proche ;
ladite étiquette de communication en champ proche est agencée sur la surface dudit logement, au niveau d'une position plus proche de ladite deuxième surface que de ladite première surface et plus proche d'un côté opposé dudit premier sommet de ladite deuxième surface que dudit premier sommet de ladite deuxième surface ; dans lequel
ladite ouverture est formée sur ladite surface latérale, et
ladite étiquette de communication en champ proche est agencée au niveau d'une position plus proche du centre dudit côté opposé que dudit premier sommet sur ladite deuxième surface dudit logement ;
ladite section de chargement de cartouches dudit dispositif de présentation d'arômes a une forme intérieure symétrique dans le sens de la rotation autour d'un centre prescrit, et reçoit chaque dite cartouche d'arôme de telle sorte que ledit premier sommet est positionné de manière adjacente par rapport audit centre et de telle sorte que lesdites deuxièmes surfaces de ladite pluralité de cartouches d'arôme sont alignées sur un plan ;
ledit dispositif de présentation d'arômes comportant par ailleurs
un étage de puces servant à des fins de montage d'une pluralité de puces de communication en champ proche agencées au niveau de positions permettant la communication avec ladite étiquette de communication en champ proche de l'une quelconque de ladite pluralité de cartouches d'arôme, correspondant respectivement à ladite pluralité de cartouches d'arôme chargées dans ladite section de chargement de cartouches, dans lequel
chacune de ladite pluralité de puces de communication en champ proche est agencée au niveau d'une position plus proche du centre du côté opposé dudit premier sommet que dudit premier sommet de ladite deuxième surface de l'une de ladite pluralité de cartouches d'arôme.

10. Dispositif de présentation d'arômes selon la revendication 9, dans lequel chacune desdites puces de communication en champ proche reçoit, quand ladite cartouche d'arôme est chargée au niveau d'une partie proche de ladite puce de communication en champ proche, des données identifiant la source de parfum contenue dans ladite cartouche d'arôme par la communication en champ proche avec ladite étiquette de communication en champ proche de ladite cartouche d'arôme, et émet en sortie les données identifiant la source de parfum et les données identifiant ladite étiquette de communication en champ proche ;
ledit dispositif de présentation d'arômes comportant par ailleurs un circuit de commande recevant lesdites données identifiant la source de parfum en provenance de chacune desdites puces de communication en champ proche, et en se basant sur les données, entraînant cette cartouche d'arôme qui a une source de parfum souhaitée à émettre le parfum.

11. Dispositif de présentation d'arômes selon la revendication 10, dans lequel les puces de ladite pluralité de puces de communication en champ proche sont agencées sur un cercle centré au niveau dudit centre prescrit.

12. Dispositif de présentation d'arômes selon la revendication 11, dans lequel les puces de ladite pluralité de puces de communication en champ proche sont agencées sur ledit cercle à équidistance les unes par rapport aux autres.

13. Dispositif de présentation d'arômes selon la revendication 12, dans lequel ledit cercle est sur le côté opposé à ladite première surface par rapport audit plan sur lequel lesdites deuxièmes surfaces sont alignées.

14. Système de dispositif de présentation d'arômes comportant une cartouche ou une pluralité de cartouches d'arôme et un dispositif de présentation d'arômes émettant un parfum en provenance de la pluralité de cartouches d'arôme ; dans lequel
chacune de ladite pluralité de cartouches d'arôme comprend
un logement en forme de colonne ayant des première et deuxième surfaces qui, chacune, ont sensiblement une forme de triangle et qui sont parallèles et congruentes l'une par rapport à l'autre et une surface latérale qui relie les première et deuxième surfaces ;
ledit logement a une partie creuse servant à stocker une source de parfum, un passage pour parfum s'ouvrant au niveau de ladite première surface servant à émettre un parfum en provenance de la source de parfum dans la partie creuse jusqu'à l'extérieur, raccordant ladite partie creuse à l'extérieur, et une ouverture servant à introduire de l'air en provenance de l'extérieur jusqu'à ladite partie creuse ;
chacune desdites première et deuxième surfaces a un premier sommet correspondant ;
ledit passage pour parfum est ouvert au niveau d'une partie proche dudit premier sommet de ladite première surface ;
ladite cartouche d'arôme comprend par ailleurs
une étiquette de communication en champ proche mise en oeuvre sur ladite deuxième surface ou sur ladite surface latérale dudit logement servant à transmettre des données d'identification pour identifier le type de la source de parfum contenue dans la cartouche d'arôme à une puce de communication en champ proche par la communication en champ proche ;
ladite étiquette de communication en champ proche est agencée sur la surface dudit logement, au niveau d'une position plus proche de ladite deuxième surface que de ladite première surface et plus proche d'un côté opposé dudit premier sommet de ladite deuxième surface que dudit premier sommet de ladite deuxième surface ; dans lequel
ladite ouverture est formée sur ladite surface latérale, et
ladite étiquette de communication en champ proche est agencée au niveau d'une position plus proche du centre dudit côté opposé que dudit premier sommet sur ladite deuxième surface dudit logement ;
ledit dispositif de présentation d'arômes a une section de chargement de cartouches servant à contenir une pluralité de cartouches d'arôme ;
ladite section de chargement de cartouches a une forme intérieure symétrique dans le sens de la rotation autour d'un centre prescrit, et reçoit chaque dite cartouche d'arôme de telle sorte que ledit premier sommet est positionné de manière adjacente par rapport audit centre et de telle sorte que lesdites deuxièmes surfaces de ladite pluralité de cartouches d'arôme sont alignées sur un plan ;
ledit dispositif de présentation d'arômes comprend par ailleurs un étage de puces servant à des fins de montage d'une pluralité de puces de communication en champ proche agencées au niveau de positions permettant la communication avec ladite étiquette de communication en champ proche de l'une quelconque de ladite pluralité de cartouches d'arôme, correspondant respectivement à ladite pluralité de cartouches d'arôme chargées dans ladite section de chargement de cartouches, et dans lequel
chacune de ladite pluralité de puces de communication en champ proche est agencée au niveau d'une position plus proche du centre du côté opposé dudit premier sommet que dudit premier sommet de ladite deuxième surface de l'une de ladite pluralité de cartouches d'arôme.
